# EUROPEAN PATENT APPLICATION

(11) **EP 2 052 684 A1**
(43) Date of publication of application: **29.04.2009**
(21) Application number: 09001585.0
(22) Date of filing: 17.03.2004
(51) Int. Cl.: A61B 5/15

(54) **Puncture device**

(30) Priority: 17.03.2003 JP 2003071516
(62) Divisional of application: 04721375.6
(71) Applicant: ARKRAY INC., Kyoto-shi, Kyoto 601-8045 (JP)
(72) Inventor: Sakata, Tetsuya, Kyoto-shi Kyoto 6018045 (JP); Matsumoto, Daisuke, Kyoto-shi Kyoto 6018045 (JP); Komuro, Hidefumi, Kyoto-shi Kyoto 6018045 (JP)
(74) Representative: Merrifield, Sarah Elizabeth

(57) **Abstract**

The present invention relates to a lancing device (1C) that includes a moving member (4) for moving a needle in an advancing direction from a standby position to a puncturing position, and also includes a housing (3) allowing the moving member (4) to move in the advancing and retreating direction. The lancing device (1C) is provided with a dividing wall (80B) for dividing an inner space of the housing (3) into a first space (35) and a second space. The lancing device (1C) moves the moving member (4) in the retreating direction to the standby position by a pressure difference between the first space (35) and the second space.

## Description

### TECHNICAL FIELD

The present invention relates to a device for sticking a needle into the skin to obtain a sample of body fluid such as blood or a tissue.

### BACKGROUND ART

For obtaining a sample of blood from skin, use may be made of a lancing device which includes a needle held by a moving member and a housing accommodating the moving member, the moving member being operated to advance the needle for puncturing the skin (see JP-A-H11-206742 and JP-A-2001-515377, for example). For promoting bleeding after the puncturing, a lancing device applies negative pressure to the skin to make the skin congested and to widen the wound caused by the puncturing (see JP-A-2001-515377).

As shown in Fig. 8, a lancing device disclosed in JP-A-H11-206742 includes a needle 91 held by a moving member 90 which is advanced by a spring. In the lancing device 9A, when the moving member 90 is at a standby position, the moving member 90 engages with a housing 92 and a coil spring 93 is compressed. To advance the moving member 90, the moving member 90 is released from the engagement with the housing 92, and then the resilient force of the coil spring 93 is applied to the moving member 90, thereby advancing the moving member 90 and the needle 91.

With the lancing device 9A, a user needs to engage the moving member 90 with the housing 92 to bring the moving member 90 to the standby position. This operation is troublesome and can be a heavy burden on the user.

As shown in Fig. 9A, the lancing device disclosed in JP-A-2001-515377 includes a needle 95 held by a moving member 94 which is advanced by pressure difference. In the lancing device 9B, when the moving member 94 is at a standby position, the space 92, in which the moving member 94 is held, is in a decompressed state, while the moving member 94 is supported by restoring force by a coil spring 97 (or bellows (not shown)). The lancing device 9B further includes a contact portion 98 for contacting with skin S. The inside of the contact portion 98 is decompressed to apply negative pressure to the skin S. As shown in 9B, to advance the moving member 94 and the needle 95, the outside air is introduced into the space 96 to increase the pressure in the space 96. In this way, pressure difference is caused between the space 96 and the inside of the contact portion 94, and the pressing force due to the pressure difference is applied to the end of the moving member 94. Then, the pressing force advances the moving member 94 and the needle 95.

With the lancing device 9B, the user needs not to move the moving member 94 to bring the moving member 94 into the standby position, whereby the burden of the user is reduced. However, if the contact portion 98 of the lancing device 9B does not intimately contact with the skin S, the air may flow into the contact portion 98 from between the skin S and the tip end of the contact portion 98. In this situation, the pressure difference between the inside of the contact portion 98 and the space 96 is reduced, and accordingly the pressing force applied to the moving member 94 is reduced. As a result, the speed of the moving member 94 and the needle 95 for puncturing is reduced. Further, since the contact state between the skin S and the tip end of the contact portion 98 depends on weather the skin S is hairy or not, there may be a variation in the amount of the air flow into the contact portion 98. In light of these, the pain resulting from the puncturing can be unbearable, depending on the punctured portion or the amount of the air flowing into the contact portion 98.

### DISCLOSURE OF THE INVENTION

An object of the present invention is to reduce the load of the user as well as to reduce pain of the patient on sampling body fluid or a tissue.

A lancing device according to a first aspect of the present invention comprises a moving member for moving a needle in an advancing direction from a standby position to a puncturing position, and also comprises a housing allowing the movement of the moving member in the advancing direction and in a retreating direction opposite to the advancing direction. The moving member moves in close contact with the housing. The housing includes a first space which is offset in the retreating direction from a portion contacting with the moving member, and a second space offset in the advancing direction from the portion contacting with the moving member. When the moving member is brought into the standby position, the moving member is moved in the retreating direction by causing a pressure difference between the first space and the second space.

The lancing device further comprises a fixing means for fixing the moving member to the housing at the standby position, with an urging force applied in the advancing direction. The device also comprises a disengaging means for dissolving the fixing of the moving member. Preferably, the moving member is moved by the urging force in the advancing direction from the standby position.

The urging force may be applied to the moving member by a resilient member. The resilient member is typically a coil spring or a bellows, and is made of foam or rubber, for example.

The lancing device according to the present invention moves the moving member to the standby position by utilizing the pressure difference resulting in a suctioning force to the moving member in the retreating direction. The suctioning force is applied to the moving member by making the pressure in the first space smaller than the pressure in the second space beyond a predetermined value. Preferably, the pressure in the first space may be made smaller than the atmospheric pressure by a predetermined value.

The lancing device according to the present invention further comprises a negative pressure generating means for generating a negative pressure in the second space.

The negative pressure generating means may apply negative pressure to the first and the second space individually. The negative pressure generating means may apply the negative pressure to the first space for causing a suctioning force to act on the moving member, thereby moving the moving member toward the standby position.

The negative pressure generating means is typically an electrical pump. Alternatively, The negative pressure generating means may be a manually operable pump.

In the lancing device according to the present invention, air flow into the first space is caused before or on disengaging the moving member by the disengaging means. The air flow into the first space is caused when the moving member is disengaged by the disengaging means.

Preferably, the disengaging means comprises an operating portion to be operated to cause the disengaging means to act on the engaging means. The positional selection of the operating portion determines whether the first space is caused to communicate with outside or not to communicate with the outside.

Preferably, the operating portion is movable in the advancing direction and the retreating direction, with part thereof protruding out of the housing. The operating portion includes an engaging part accommodated in the housing. The housing is formed with a through-hole for allowing the movement of the operating portion in the advancing direction and in the retreating direction. Preferably, the engaging part is used to select between a state in which the engaging portion closes the through-hole and another state in which the engaging portion does not close the through-hole.

In the lancing device according to the present invention, the second space is provided with a retreating means for moving the needle back in the retreating direction after the needle is brought to the puncturing position.

According to a second aspect of the present invention, a lancing device comprises a moving member for moving a needle in an advancing direction from a standby position to a puncturing position, and also comprises a housing for allowing the movement of the moving member in the advancing direction and in a retreating direction opposite to the advancing direction. A dividing wall is provided for dividing an inner space of the housing into a first space offset in the retreating direction and a second space offset in the advancing direction. When the moving member is brought into the standby position, the moving member is moved in the retreating direction by causing a pressure difference between the first space and the second space.

The dividing wall may include a bellows.

The lancing device according to the present invention further comprises a fixing means for fixing the moving member to the housing at the standby position, with an urging force applied in the advancing direction. The moving member is moved by the urging force in the advancing direction from the standby position.

Preferably, the urging force is applied to the moving member by at least one resilient member.

Preferably, the resilient member may be the bellows mentioned above, or may be a coil spring. The lancing device according to the present invention may apply the urging force to the moving member by the bellows serving as the dividing wall. When the bellows serves as the dividing wall, the bellows may not be arranged to apply urging force to the moving member, but may serve only as the dividing wall.

### BRIEF DESCRIPTION OF THE DRAWINGS

Fig. 1 is a sectional view illustrating an example of a lancing device according to the present invention.
Fig. 2A is a sectional view illustrating a latching operation of a lancet holder, and Fig. 2B is a sectional view illustrating an attaching operation of a lancet.
Fig. 3 is a sectional view illustrating a raising operation of the skin.
Figs. 4A and 4B are sectional views illustrating a releasing operation of the latching and the air flow into a decompression space.
Fig. 5 is a sectional view illustrating the puncture operation.
Fig. 6 is a sectional view illustrating another example of the lancing device according to the present invention.
Fig. 7 is a sectional view illustrating still another example of the lancing device according to the present invention.

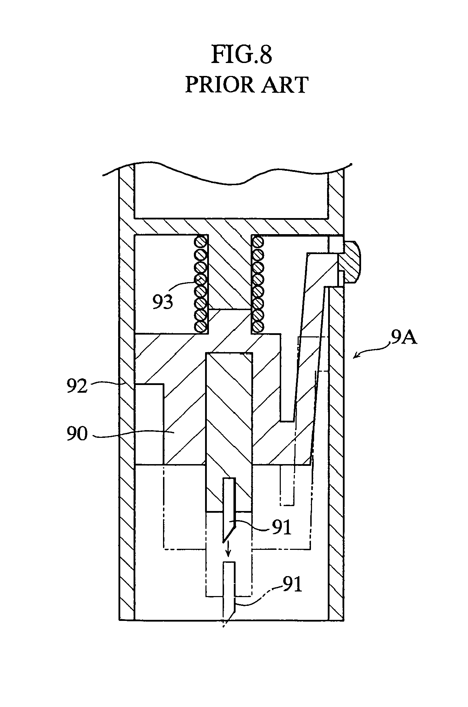

Fig. 8 is a sectional view illustrating a principal part of a conventional lancing device.

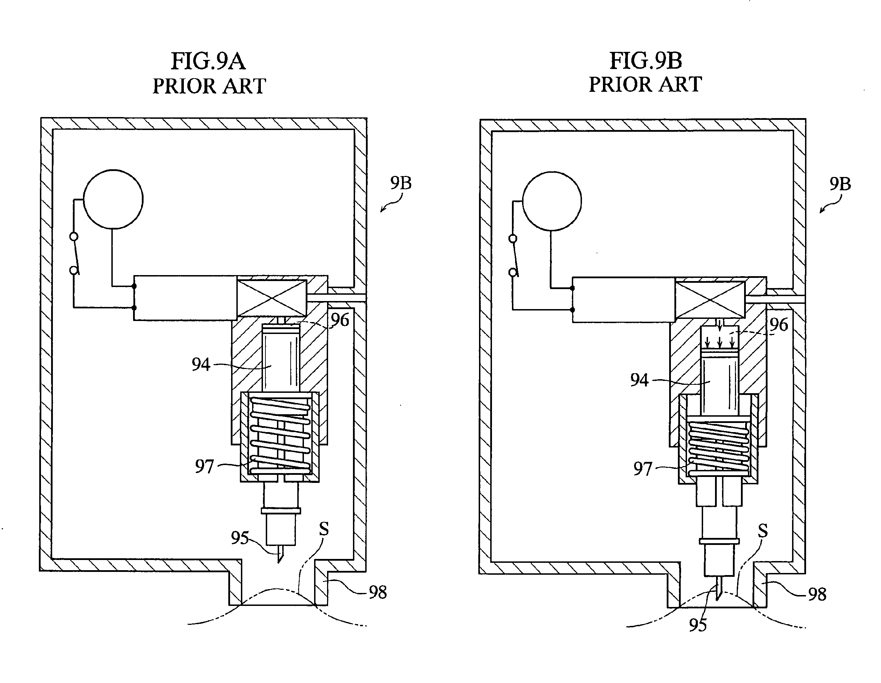

Figs. 9A and 9B are sectional views illustrating another example of the conventional lancing device.

### BEST MODE FOR CARRYING OUT THE INVENTION

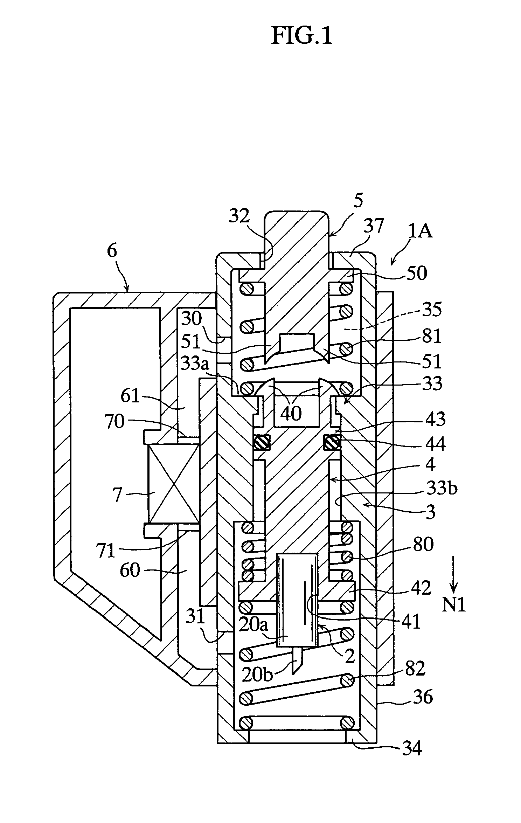

As shown in Fig. 1, a lancet 2 is attached to a lancing device 1A in use. The lancet 2 includes a body 20a and a needle 20b protruding from the body. The needle 20b is made of e.g. metal, and the body 20a is made of e.g. synthetic resin. The needle 20b may be integrally embedded in the body 20a when the body is produced by insert molding, for example.

The lancing device 1A includes a housing 3, a lancet holder 4, an operating cap 5, a case 6, and an electrical pump 7.

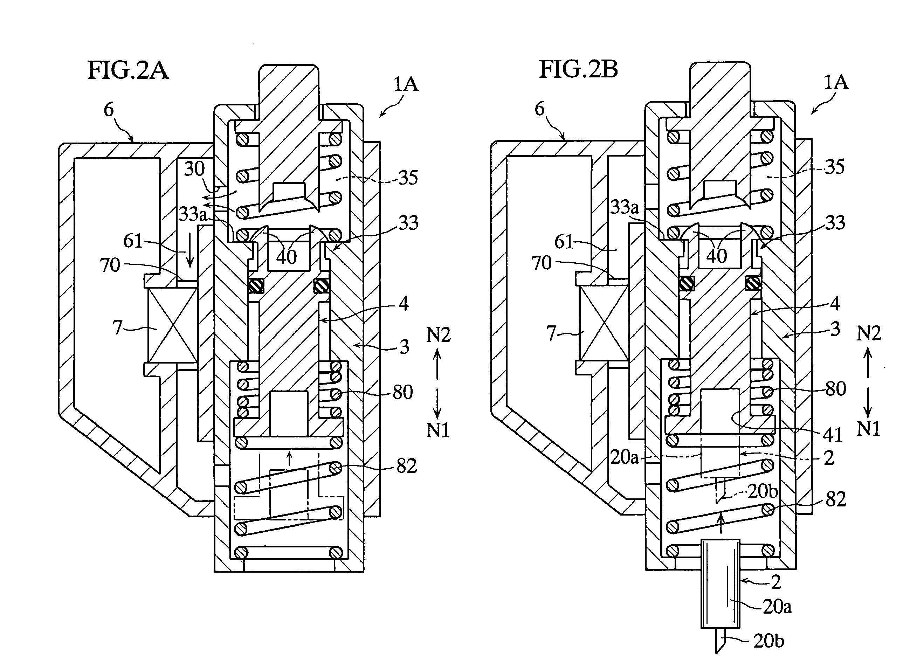

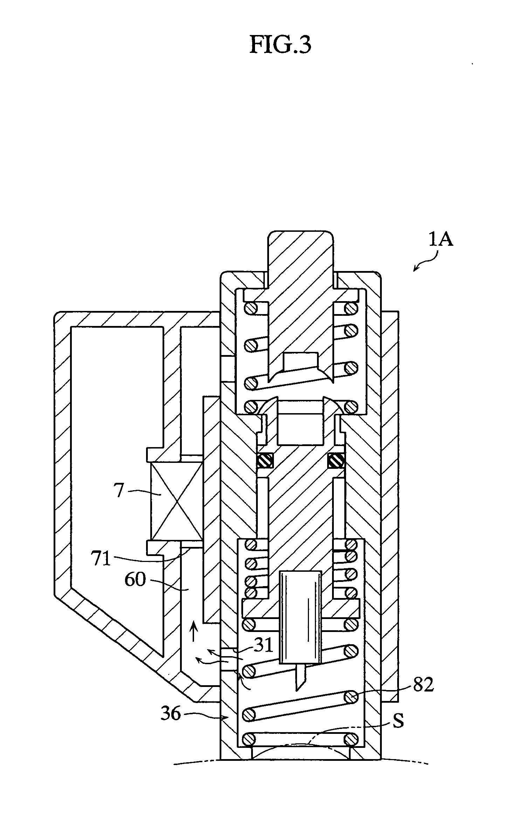

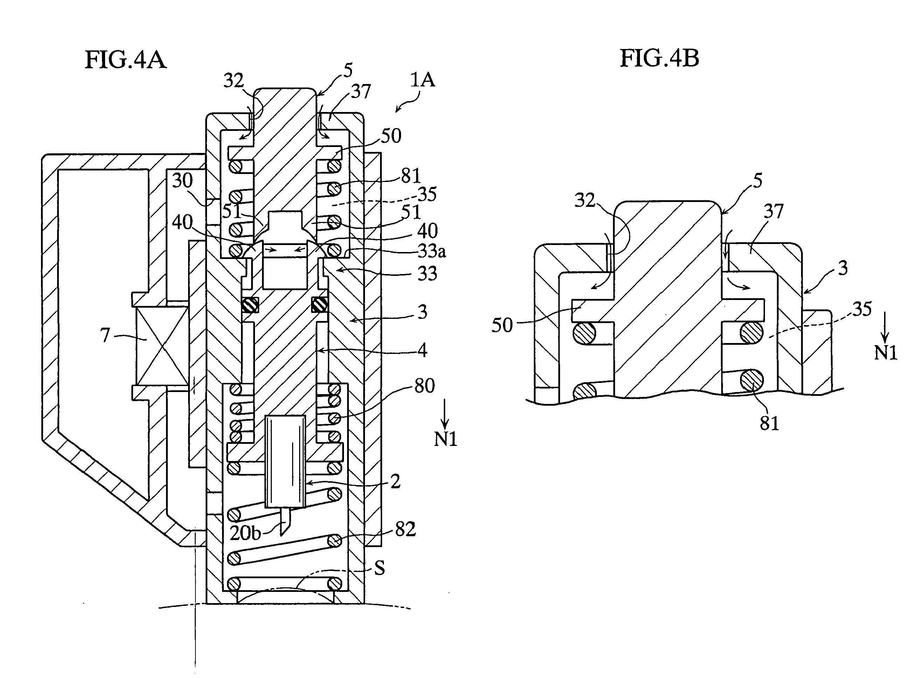

The housing 3 accommodates the lancet holder 4. The housing 3 includes through-holes 30, 31, 32, projections 33, 34, a decompression space 35, and a contact portion 36. As shown in Fig. 2A, the through-hole 30 is used for discharging the air out of the decompression space 35. As shown in Fig. 3, the through-hole 31 is used for discharging the air out of the contact portion 36. As seen from in Figs. 1-4A, 4B, the through-hole 32 allows the movement of the operating cap 5, and is used for introducing the air into the decompression space 35.

As shown in Fig. 1, the projection 33 engages with the lancet holder 4 and coil springs 80, 81. The projection 34 provided at the contact portion 36 engages with a coil spring 82.

As shown in Fig. 2A, the decompression space 35 is decompressed when the lancet holder 4 is engaged with the projection 33. The decompression space 35 is decompressed by discharging the air through the through-hole 30.

As shown in Fig. 3, the contact portion 36 comes into contact with skin S to be punctured. The inner space of the contact portion 36 is under negative pressure on puncturing so that the skin S is raised. The negative pressure is generated by discharging the air out of the contact portion 36.

As shown in Figs. 1-5, the lancet holder 4, for holding the lancet 2, is advanced toward a tip of the housing 3 (in the N1 direction) by pressing the operating cap 5. The lancet holder 4 is formed with a pair of claws 40, a recess 41, and flanges 42, 43.

As shown in Figs. 1 and 4A, claws 40 can engage with an upper surface 33a of the projection 33 of the housing 3, and are resiliently movable toward and away from each other. As shown in Fig. 1, the recess 41 holds the lancet 2. The coil spring 80 is arranged between the flange 42 and the projection 33 of the housing 3, while the coil spring 82 is arranged between the flange 42 and the projection 34. When the claws 40 is in engagement with the projection 33, the coil spring 80 is compressed. Thus, when the claws 40 are disengaged from the projection 33, the lancet holder 4 is advanced by the restoring force of the coil spring 80. When the lancet holder 4 advanced, the coil spring 82 is compressed, and then the restoring force of the coil causes the lancet holder 4 to retreat. The coil spring 82, however, may be dispensed with. The flange 43 contacts with an inner surface of the housing 3 (an inner surface of the projection 33) when the lancet holder 4 moves. The flange 43 is provided with an O-ring 44.

As shown in Figs. 1, 4A, 4B and 5, the operating cap 5 for advancing the lancet holder 4 is held in the housing 3 in a manner such that a portion of the cap protrudes from the through-hole 32. The operating cap 5 is provided with a flange 50 and a pair of pressing portions 51.

The flange 50 causes the decompression space 35 of the housing 3 to communicate with the outside via the through-hole 32 or not to communicate with the outside. The lower surface of the flange 50 contacts with the upper end of the coil spring 81. The lower end of the coil spring 81 contacts with the upper surface 33a of the projection 33 of the housing 3. In other words, the coil spring 81 is arranged between the flange 50 and the projection 33. With such an arrangement, in the normal state shown in Fig. 1, the restoring force of the coil spring 81 presses the flange 50 of the operating cap 5 toward an upper wall 37 of the housing 3, thereby closing the through-hole 32. When the operating cap 5 is pressed toward the N1 direction in the above state, the flange 50 advances to compress the coil spring 81, as shown in Fig. 4A. Conversely, when the operating cap 5 is released from the pressing force, the operating cap 5 is returned to the normal position by the restoring force of the coil spring 81, as shown in Fig. 1.

When the operating cap 5 is advanced in the N1 direction at a predetermined distance, the pressing portions 51 press the claws 40, so that each end of the claws 40 comes closer to each other. In this state, the engagement (latching) of the claws 40 with the projection 33 is released, then as described above, the lancet holder 4 is advanced by the restoring force of the coil spring 80.

As shown in Fig. 1, the case 6 holds the housing 3 and the electrical pump 7. The case 6 is formed with paths 60, 61 for communicating the electrical pump 7 with the inside of the decompression space 35 and the contact portion 36 of the housing 3.

The electrical pump 7 is controlled by a non-illustrated control means for discharging the air of the decompression space 35 and the contact portion 36 through the through-holes 30, 31 and the paths 60, 61. The electrical pump 7 includes a suctioning portion 70 for suctioning the air of the decompression space 35 through the through-hole 30 and the path 61, and a suctioning portion 71 for suctioning the air of the contact portion 36 through the through-hole 31 and the path 60, though the function is not illustrated. The control means of the electrical pump 7 switches between the suctioning state and the non-suctioning state of each of the suctioning portions 70, 71 individually.

On puncturing the skin S by the lancing device 1A, the claws 40 of the lancet holder 4 are brought into engagement or into latching with the upper surface 33a of the projection 33 of the housing 3, as shown in Fig. 2A. This latching is achieved when the decompression space 35 is decompressed by suctioning the air of the decompression space 35 at the suctioning portion 70 of the electrical pump 7 through the through-hole 30 and the path 61. Specifically, as the end of the contact portion 36 is open, the inner pressure of the contact portion 36 is the same as the atmospheric pressure. In this state, decompression of the decompression space 35 causes a pressure difference between the contact portion 36 and the decompression space 35. This pressure difference is increased as the decompression space 35 is decompressed, and works as a suctioning force to move the lancet holder 4 upwardly (in the N2 direction). When the suctioning force applied to the lancet holder 4 becomes larger than the sum of the resistance force of the coil spring 80 and the transfer resistance of the lancet holder 4 against the housing 3, the lancet holder 4 can be moved upwardly (in the N2 direction). In such an instance, the restoring force of the coil spring 82 supports upward movement of the lancet holder 4. Finally, when the lancet holder 4 moves a predetermined distance in the N2 direction, the claws 40 engages with the upper surface 33a of the projection 33 of the housing 3 to achieve the latching of the lancet holder 4.

Next, as shown in Fig. 2B, the lancet 2 is attached to the lancet holder 4. The attachment of the lancet 2 to the lancet holder 4 is performed by fitting the body 20a of the lancet 2, at the portion opposite to the needle 20b, to the recess 41.

Thereafter, as shown in Fig. 3, the contact portion 36 of the lancing device 1A is brought into contact with the skin S, and the skin S is raised by the negative pressure generated in the contact portion 36. The negative pressure is generated by suctioning the air of the contact portion 36 from the suctioning portion 71 of the electrical pump 7 through the through-hole 31 and the path 60.

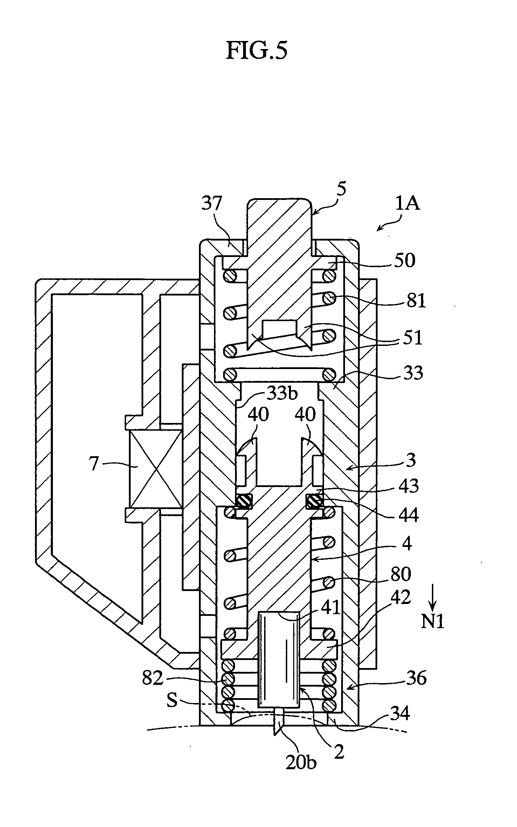

Subsequently, as shown in Figs. 4A, 4B and 5, the lancet 2 is advanced in the N1 direction to puncture the skin S by the needle 20b. On puncturing the skin S by the needle 2b, as shown in Figs. 4A and 4B, the operating cap 5 is pressed in the N1 direction to release the lancet holder 4 from latching.

When the operating cap 5 is pressed in the N1 direction, the flange 50 of the operating cap 5 is spaced from the upper wall 37 of the housing 3, whereby the air flows into the decompression space 35 through the through-hole 32 of the housing 3. Thus, the pressure in the decompression space 35 is increased and the pressure difference between the decompression space 35 and the contact portion 36 is reduced, thereby reducing the suctioning force applied to the lancet holder 4. As a result, the operating cap 5 can be lowered smoothly.

Inthisway, the operating cap 5 is moved in the N1 direction over a predetermined distance to inwardly move the claws 40 toward each other, so that the claws 40 are disengaged from the upper surface 33a of the projection 33, as shown in Fig. 4A. Here, the restoring force of the coil spring 80 advances the lancet holder 4 in the N1 direction, and then the needle 20b of the lancet 2 punctures the skin S. After the needle 20b punctures the skin S, the lancet holder 4 retreats in the N2 direction due to the restoring force of the coil springs 80, 82, thereby immediately pulling out the needle 20b from the skin S. The negative pressure applied to the skin S causes the bleeding from the portion which is punctured by the needle 20b.

The lancing device 1A utilizes the electrical pump 7 to apply the suctioning force to the lancet holder 4 so that the latching of the lancet holder 4 is achieved. Thus, the lancet holder 4 can be latched by an easy operation such as pressing operating buttons provided at the lancing device 1A, for example. Such structure facilitates troublesome operation for latching the lancet holder 4. Further, the lancing device 1A utilizes the restoring force of the coil spring 80 to advance the lancet holder 4 in the N1 direction. Thus, the lancet holder 4 can be moved at a constant speed, without depending on the inner pressure of the contact portion 36. Due to the structure, even if the air flows into the contact portion 36 and thus the inner pressure of the contact portion 36 is increased, the lancet holder 4 and the needle 20b can be advanced as desired.

In the above description, the negative pressure is applied to the contact portion 36 before the needle 20b punctures the skin S. However, it may be applied to the skin S after the needle 20b punctures the skin S. The decompression space 35 and the contact portion 36 may be decompressed by utilizing a manual pump, in place of the electrical pump 7. The air flow into the decompression space 35 may not necessarily be caused by pressing the operating cap 5. An operating button other than the operating cap 5 may be used to cause the air flow into the decompression space 35. The lancet holder 4 may be latched by increasing the inner pressure of the contact portion 36 and applying pressing force upwardly (in the N1 direction) from the contact portion 36 toward the lancet holder 4.

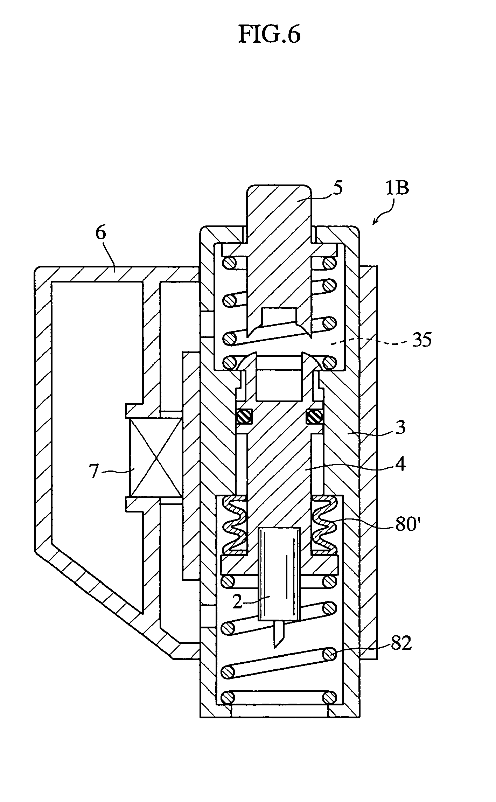

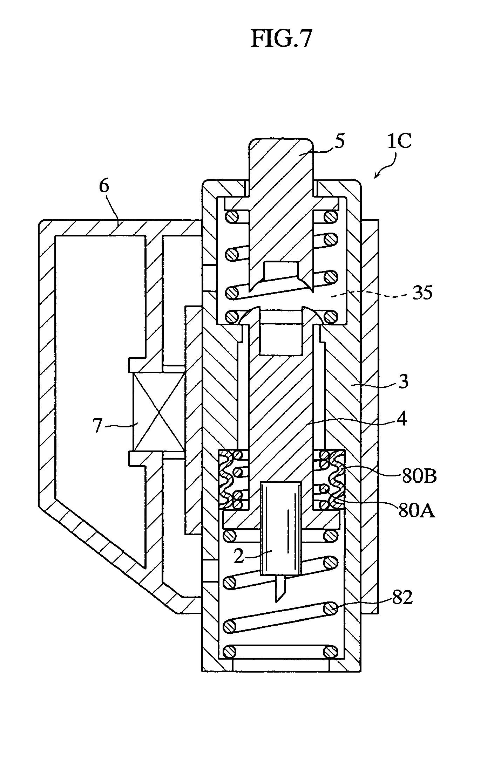

In the present embodiment, the lancing device utilizes the restoring force of the coil spring for advancing the lancet holder. However, as in the lancing device 1B shown in Fig. 6, the coil spring may be replaced with a bellows 80'. As in the lancing device 1C shown in Fig. 7, both a coil spring 80A and a bellows 80B may be used. In the lancing device 1C, differently from the lancing devices 1A, 1B (see Figs. 1 and 6), the lancet holder 4 does not move in contact with the housing 3, but the bellows 80B serves as a dividing wall to define the decompression space 35. Of course, the lancing device 1B (see Fig. 6), using the bellows 80' in place of the coil spring, may also be designed so that the lancet holder 4 doesn't move in contact with the housing 3 but the bellows 80' serves as a dividing wall to define the decompression space 35.

The present invention is applicable to a lancing device having a measuring function in addition to the puncturing function. This lancing device may be provided with a measuring tool such as a biosensor. The present invention is applicable to a lancing device which causes the skin to bleed, supplies the blood to the measuring tool, and measures the concentration of glucose, cholesterol, or lactic acid in the blood, for example.

## Claims

1. A lancing device comprising:
a moving member for moving a needle in an advancing direction from a standby position to a puncturing position; and
a housing allowing the moving member to move in the advancing direction and in a retreating direction opposite to the advancing direction,
wherein a dividing wall is provided for dividing an inner space of the housing into a first space offset in the retreating direction and a second space offset in the advancing direction,
wherein the moving member is moved in the retreating direction to be brought to the standby position by a pressure difference produced between the first space and the second space.

2. The lancing device according to claim 1, wherein the dividing wall includes a bellows.

3. The lancing device according to claim 2, further comprising a fixing means for fixing the moving member to the housing at the standby position, with an urging force applied in the advancing direction,
wherein the moving member is moved from the standby position in the advancing direction by the urging force.

4. The lancing device according to claim 3, wherein the urging force is applied to the moving member by at least one resilient member.

5. The lancing device according to claim 4, wherein said at least one resilient member comprises the bellows.

6. The lancing device according to claim 5, wherein said at least one resilient member further comprises a coil spring.
